# EUROPEAN PATENT SPECIFICATION

(11) **EP 2 654 891 B1**
(45) Date of publication and mention of the grant of the patent: **18.11.2020**
(21) Application number: 11811583.1
(22) Date of filing: 21.12.2011
(51) Int. Cl.: A61N 5/00, A61N 5/10

(54) **A MOBILE X-RAY UNIT AND METHOD**
MOBILE RÖNTGENEINHEIT UND VERFAHREN
EQUIPEMENT RADIOLOGIQUE MOBILE ET MÉTHODE

(30) Priority: 22.12.2010 NL 2005899; 23.12.2010 US 201061426925 P
(43) Date of publication of application: 30.10.2013
(73) Proprietor: Nucletron Operations B.V., 3905 TH Veenendaal (NL)
(72) Inventor: VAN DER VEEN, Johannes Simon, 3905 TH Veenendaal (NL); WOUDSTRA, Bas, 3905 TH Veenendaal (NL); HENNING, Johan, 3905 TH Veenendaal (NL)
(74) Representative: V.O.
(86) International application number: PCT/NL2011/050878
(87) International publication number: WO 2012/087131

(56) References cited:
- EP-A2- 1 308 185
- WO-A1-95/04501
- WO-A2-2004/103145
- US-A1- 2003 150 983
- US-A1- 2004 013 240
- US-A1- 2007 165 779
- US-B1- 6 241 670
- Topex, Inc: "SRT 100 Superficial Radiotherapy System for the Treatment of Skin Cancer", , 31 December 2007 (2007-12-31), XP002656846, Retrieved from the Internet: URL:http://www.rsllabin.com/pdf/TOPEXBROCH URE_v9.pdf [retrieved on 2011-08-18]
- Topex, Inc: "Regulatory Information", , 31 December 2007 (2007-12-31), XP002656847, Retrieved from the Internet: URL:http://www.topexmedical.com/product2.h tml [retrieved on 2011-08-18]
- Nancy C Brogdon: "5. 510(k) Summary", , 1 January 2004 (2004-01-01), XP055346297, Retrieved from the Internet: URL:https://www.accessdata.fda.gov/cdrh_do cs/pdf6/K063456.pdf [retrieved on 2017-02-15]
- Topex ET AL: "TOPEX and Therapy Remarketing Group Expand Distribution for Skin Cancer Treatment System", , 16 September 2008 (2008-09-16), XP055350017, Retrieved from the Internet: URL:http://www.prnewswire.com/news-release s/topex-and-therapy-remarketing-group-expa nd-distribution-for-skin-cancer-treatment- system-65116392.html [retrieved on 2017-02-28]
- Anonymous: "TOPEX", , 30 January 2008 (2008-01-30), XP055346502, Retrieved from the Internet: URL:http://web.archive.org/web/20080130220 208/http://www.topexmedical.com/product1.h tml [retrieved on 2017-02-16]

## Description

### FIELD OF THE INVENTION

The invention relates to a mobile X-ray unit comprising a base for accommodating a control unit and a power supply and further comprising an articulated displaceable arm supporting an X-ray applicator having an X-ray tube for emitting an X-ray beam through an exit window for irradiating an object.

The invention further relates to a method for dosimetry control of an X-ray beam emanating from the mobile X-ray unit.

### BACKGROUND OF THE INVENTION

Skin cancer, having increased incidence rate in the last decade of the 20th century, requires substantial effort from medical professionals in terms of early diagnosis, logistics and availability of suitable treatment. However, it is appreciated that over 1.3 million new skin cancers are diagnosed annually and are increasing at a rate of about 5 % per year. Increased exposure to the sun without skin protection and a decreased ozone layer are regarded as the main causes of this increase - a problem estimated to be costing over 1 billion Euros in annual medical treatment expenses. Over 80% of skin cancers occur in the head and neck regions with 50% occurring in patients over 60 years of age. It is expected that a portion of the senior population will double in year 2025 compared to the present demographics.

Non proliferated cancers being substantially superficial lesions may be treated in different ways. First, surgery may be envisaged. However, such technique may be disadvantageous in terms of long waiting lists and complications related to post-treatment care. In addition, due to invasive character of surgery contamination of the wound by infections may present an additional risk. Secondly, irradiation using electrons of soft X-rays may be envisaged. Such techniques have an advantage of being non invasive, wherein a treatment session may be as short as 2 minutes. It will be appreciated that usually the integral treatment using a radiotherapeutic technique may comprise a number of sessions.

Accordingly, the growing incidence of skin cancer and increasing of a share of the senior population in overall demographics pose substantial challenge on the cancer treatment logistics.

Recently, the use of a portable X-ray unit has been suggested, which may be used inside a hospital radiotherapy department. An embodiment of such portable unit is described in US 2007/0076851. The known unit comprises an X-ray applicator comprising X-ray source provided with a filtering device having a plurality of filters rotatably arranged with respect to a focal point of the X-ray tube for changing filtering characteristics on demand. The plurality of filters is arranged in a filtering device, which is transversely arranged with respect to a longitudinal axis of the X-ray tube. Such arrangement requires additional measure for delivering the X-ray beam towards the filter plane. The known device is used by positioning the X-ray applicator at some distance from the patient's skin. Another mobile x-ray unit is present in US6241670 and the SRT 100 from Topex.

It is a disadvantage of the known X-ray tube that poor control is available regarding actual delineation between the X-ray beam emanating from the X-ray applicator and a treated region on the patient.

### SUMMARY OF THE INVENTION

The invention is defined by the independent claims.

It is an object of the invention to provide an improved mobile X-ray. More in particular, it is an object of the invention to provide the mobile X-ray unit wherein the X-ray beam may be delivered in a controlled way.

To this end in the mobile X-ray unit according to the invention comprises a phantom-based dosimetry system adapted to perform an on-line or a real-time dosimetry check of the X-ray beam.

It will be appreciated that the terms 'mobile' and 'portable' in the context of the present application may be interchanged as these terms equally relate to an easily moved or transported device, for example, a device which may be moved or transported by a single individual

It is found to be advantageous to provide a phantom-based dosimetry system for enabling a fast and a robust means of quality assurance and radiation safety control of the X-ray unit.

Preferably, the phantom-based dosimetry system comprises a premanufactured, preferably solid, phantom block provided with one or more spaces for accommodating a suitable dose meter. It will be appreciated that the dose meter may be a film, an ionization chamber or a semiconductor detector. For facilitating film dosimetry, the phantom may be provided with extendable drawers for accommodating one or more film portions at their prescribed dwell positions. For the ionization chamber and the semiconductor device, the phantom may comprise dedicated cavities fit for a dimension of the dose meter conceived to be used. The phantom may be preferably manufactured from a material with properties corresponding to the dosimetric properties of a human skin. In particular embodiments the phantom may not only correspond to dosimetric properties of a human body, but also may simulate its external shape. For example, hands, neck, ear, face, breast, nose or any other non-flat surfaces may be embodied by the phantom. This is found to be particularly important for ensuring proper treatment planning and treatment delivery. More in particular, the phantom may comprise suitable inserts which may be used during pre-planning dosimetry as well as during treatment. For example, the phantom may comprise ear and nose inserts for ensuring an electronic equilibrium and a proper dose distribution when these organs are being irradiated. It is possible that such humanoid phantoms are provided in different shapes and sizes for taking care of different shapes of adults and the difference in sizes between children and adults, for example.

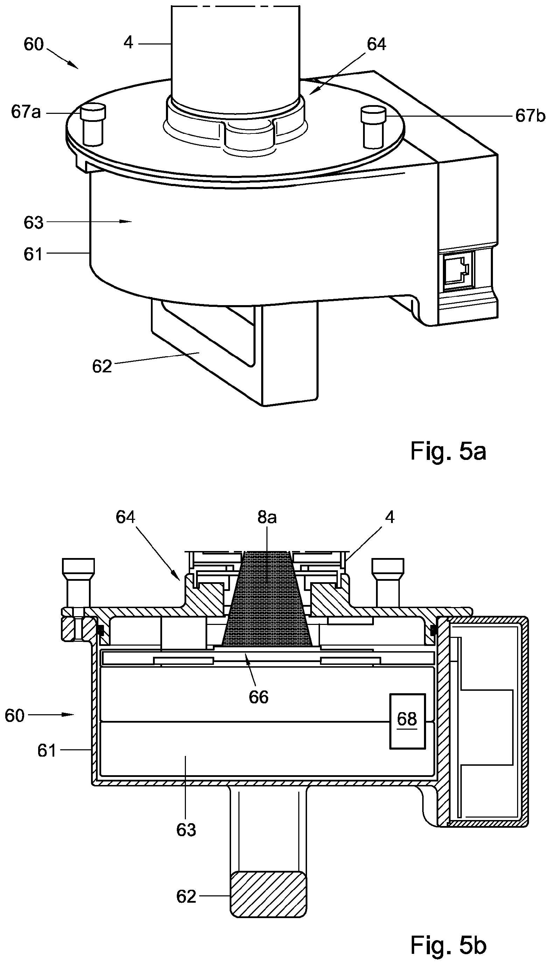

In a particular embodiment, the dosimetry system may be arranged to be connected to a receptacle of the X-ray applicator. Although a dedicated receptacle may be envisaged, preferably, the dosimetry system is connected to a collimator receptacle. For this purpose the phantom-based dosimetry system may be provided with similar attachment means as the collimators for connecting to the collimator receptacle. An embodiment of the dosimetry system connectable to the X-ray applicator according to the invention will be discussed with reference to Figures 5a and 5b.

In an embodiment of the X-ray unit the dosimetry system comprises digital read out means. It is found to be particularly advantageous to provide an on-line and/or real time dosimetry system capable of direct data transfer to a suitable data processing unit, which may be arranged in electronic communication with the controls of the X-ray unit.

In a further embodiment of the X-ray unit the dosimetry system is arranged to enable verification of at least a percentage depth dose, a beam flatness and a dose rate the of generated X-ray field.

It will be appreciated that different embodiments are contemplated. First, it is possible to dispose a dosimetric film sensitive for the X-rays generated by the X-ray tube in a direction substantially coinciding with a direction of the longitudinal axis of the emanating X-ray beam. In such way a percentage depth dose may be measured. It will be appreciated that one or more such films are possible for enabling off-center measurements.

Next, it is possible to provide a number of thermoluminiscent dosimeters (TLD) inside the phantom at pre-determined depths along a ray corresponding with a central axis of the X-ray field. Also off-center measurements may be enabled by suitably positioning the dose meters at desired locations. The irradiated TLD's may be read out by a dedicated read-out unit and the data may be made available for further analysis. For example, the TLD reader may export a computer file into a suitable data processing unit.

Next, the phantom may be provided with one or more cavities for accommodating one or more ionization chambers or one or more semiconductor devices. Such dose meters may be connected to a measurement unit capable of provided on-line dosimetry data. A signal from the measurement unit may be supplied to the control unit of the mobile X-ray unit according to the invention for recording the dosimetry data and/or for adjusting settings of a high voltage power supply, if necessary.

It will be appreciated that preferably, the set of miniature ionization chambers is used. Alternatively, the cavities may be provided with diode radiation detectors, comprising a first set positioned at the surface of the phantom based dosimetry system and a second set positioned at a depth of 5 mm. It will be appreciated that preferable individual detectors from the second set are positioned outside the shadow of the detectors from the first set. In a particular embodiment 10 - 15 detectors are provided at the depth of 5 mm and 10 - 15 detectors are provided at the depth of 10mm, in a position away from the shadow of the higher detectors. It will be appreciated that the absolute value of the first depth and the second depth for providing the detector's set may be chosen differently.

In a still further embodiment of the X-ray unit the dosimetry system is calibrated for enabling absolute dosimetry of the percentage depth dose. It is found to be particularly advantageous when the phantom-based dosimetry system is capable of delivering absolute dosimetry data on-line or in real time. Those skilled in the art will readily appreciate which calibration measures are necessary for enabling absolute calibration of a dosimetric film, an ionization chamber or a semiconductor detector.

In a still further embodiment of the mobile X-ray unit a surface of the phantom is provided with an alignment pattern.

It is found to be particularly advantageous to provide suitable alignment pattern on a surface and/or inside the phantom. It will be appreciated that to enable visualization of the internal pattern, the phantom material must be substantially transparent.

It will be further appreciated that the alignment pattern may also be used for verification of geometric correspondence between an expected X-ray field, fully or partially visualized using a light indicator and the actual X-ray field. Preferably, such correspondence is checked for different angulations of the X-ray applicator.

The dosimetry system, i.e. a film or a suitable device (TLD, an ionization chamber or a semiconductor) may comprise a plurality of measuring points, preferably distributed in a plane. When such device is positioned in the X-ray field, the readings may be processed for establishing dose data across the applied field. For example, a reading at the central axis may be taken and a number of peripheral readings, preferably at different radial distances. As a result, information may be obtained regarding not only the absolute dose in the central field, but also information about beam flatness across the field. It will be appreciated that for such purposes film dosimetry may be preferable.

In a still further embodiment of the X-ray unit it further comprises an indicator for visualizing at least a portion of the X-ray beam emanated from the exit window.

It is found that treatment efficacy is substantially improved when the indicator is provided for visually delineating at least a portion of the generated X-ray beam, like a central axis thereof, and/or a full beam geometry.

In particular, such indication may be advantageous for positioning of the phantom of the dosimetry system with respect to the X-ray beam. Preferably, the indicator comprises a light source. The light source may be arranged in the X-ray applicator or, alternatively it may be arranged around the outer surface of the X-ray applicator. In the former case the light indicator may be arranged to delineate the central axis of the X-ray beam and/or the full beam geometry, whereas in the latter case the light indicator may be arranged to delineate a central axis of the X-ray beam, preferably at a pre-determined distance from the X-ray applicator. Such a feature may be advantageous when the X-ray applicator is used at a standard distance from the patient's skin. However, it will be appreciated that the light indicator arranged around the X-ray applicator may be adjustable for indicating the central axis of the X-ray beam at a variety of axial distances from the X-ray applicator. When axial visualization is envisaged, it is particularly advantageous to use a transparent phantom provided with an internal alignment pattern for verifying a coplanar alignment between a central axis of the phantom and the central ray of the X-ray field.

Alternatively, the dosimetry system may be attached to the X-ray applicator so that the exit surface through which the X-ray beam passes in use is in contact with an upper surface of the phantom-based dosimetry system.

In an embodiment of the mobile X-ray unit the indicator comprises two or more light sources concentrically arranged around the X-ray applicator. Although it may be sufficient to provide a single light source generating a narrow beam for indicating the central axis of the X-ray beam, it is found to be advantageous to provide a plurality of light sources generating respective narrow light beams intersecting at a given distance from an exit surface of the X-ray applicator. Due to this embodiment installation of the X-ray applicator at a prescribed distance from the phantom is enabled as well as accurate installation of the dosimetry system with respect to the X-ray beam. In order to ensure a correct coverage of the target portion by the X-ray beam, the X-ray applicator may be positioned so that the indicated center of the X-ray beam is positioned substantially at a center of the phantom, which is preferably provided with an alignment pattern. It will be appreciated that such embodiment functions particularly well for regular shaped X-ray beams, for example, when a circular, a square, an elliptic, or a triangular collimator is used for shaping the X-ray beam.

In a still further embodiment of the mobile X-ray unit the indicator comprises a light source accommodated inside the X-ray applicator for generating a light beam conceived to be intercepted by the collimator for providing a light image of the X-ray field emanating from the exit surface.

This embodiment is found to be particularly advantageous when the full shape of the X-ray beam is to be delineated, for example, in situations when an irregular beam shape is used. In such case, preferably, the light source may be provided near the target or, via a mirror, off-axis, for generating a light beam conceived to be intercepted by the collimator. It will be appreciated that a direction of propagation of the light beam must be essentially conformal to a direction of propagation of the X-ray beam. In an embodiment, when a mirror is used, the light source may advantageously be positioned off-axis.

In a still further embodiment of the mobile X-ray unit the indicator comprises a light source and an optical fiber arranged to deliver light from the light source for interception by the collimator.

This embodiment has an advantage that the light source may be positioned outside the X-ray applicator for not compromising its overall size. For example, the light source may be arranged in the base of the X-ray unit and the optical fibers may run from the base to inside the X-ray applicator for suitably illuminating the collimator for obtaining a light image of the generated X-ray beam.

In a still further embodiment of the mobile X-ray unit the indicator may comprise a plurality of optical fibers distributed in the X-ray applicator in an area above the collimator for illuminating a collimator opening for causing the collimator opening to intercept the resulting light field. This embodiment may be advantageous for obtaining a light field having substantial intensity.

In a still further embodiment of the mobile X-ray unit the indicator comprises a light source emitting a narrow light beam arranged inside the applicator for delineating the longitudinal axis of the X-ray beam. Preferably, a miniature laser source is used.

In a still further embodiment of the X-ray unit a radiation detector is provided inside the outer housing for detecting the X-ray beam.

Preferably, the X-ray unit comprises a primary timer which sets a time for the high voltage supply for delivering a predetermined radiation dose. The radiation sensor accommodated inside the X-ray applicator may be part of a secondary timer circuit adapted to shut down the high voltage supply upon the predetermined radiation dose is delivered. In this way radiation safety control may be improved.

It is found to be advantageous to provide independent means for detecting presence of the generated X-ray beam. Preferably, in an embodiment when the dosimetry system is operable to provide absolute radiation dose data on-line or in real time, the signal from the dosimetry system may be used in addition to the signal from the build-in radiation detector for carrying out quality control of the built-in interrupts of the X-ray unit according to the invention.

According to an aspect there is provided a method for dosimetry control of an X-ray beam emanating from a mobile X-ray unit comprising a base for accommodating a control unit, a power supply and a cooler and further comprising an articulated displaceable arm supporting an X-ray applicator having an X-ray tube for generating an X-ray beam, the method comprising:
- providing a phantom-based dosimetry system for verifying at least a percentage depth dose of the X-ray beam.

Preferably, a solid phantom provided with one or more dose meters for checking percentage depth dose, beam flatness and the dose rate is used. The phantom may be manufactured from a material with properties corresponding to dosimetric properties of human skin. In a particular embodiment the phantom is humanoid, simulating an outer surface of a portion of a human body, such as an ear, a hand, a nose, a breast, a neck and so on.

In a further embodiment of the method an indicator is provided in or near the X-ray applicator for visually delineating at least a portion of the X-ray beam for positioning the phantom-based dosimetry system. Preferably, the indicator comprises a light source arranged to generate a light field conceived to be intercepted by a collimator opening for providing visualization of the X-ray beam. Alternatively, the indicator may comprise a light source arranged to delineate a longitudinal axis of the X-ray beam.

These and other aspects will be discussed with reference to drawings wherein like reference numerals or signs relate to like elements. It will be appreciated that the drawings are presented for illustration purposes only and may not be used for limiting the scope of the appended claims.

### BRIEF DESCRIPTION OF THE DRAWINGS

Figure 1a presents in a schematic way an embodiment of a mobile X-ray unit
Figure 1b presents in a schematic way an embodiment of a displaceable panel of the mobile X-ray unit.
Figure 1c presents in a schematic way an embodiment of displacement functionality of the applicator of the X-ray unit.
Figure 2 presents in a schematic way an embodiment of architecture of the mobile X-ray unit
Figure 3 presents in a schematic way a phantom-based dosimetry system of the X-ray unit
Figure 4a presents in a schematic way a first embodiment of a cross section of an X-ray applicator of the mobile X-ray unit depicting a first embodiment of the indicator.
Figure 4b presents in a schematic way a second embodiment of a cross section of an X-ray applicator of the mobile X-ray unit depicting a second embodiment of the indicator.
Figure 4c presents in a schematic way a third embodiment of a cross section of an X-ray applicator of the mobile X-ray unit depicting a third embodiment of the indicator.
Figure 5a presents in a schematic way an embodiment of a phantom-based dosimetry system
Figure 5b presents in a schematic way an embodiment of the phantom-based dosimetry system of Figure 5a in a cross-section.
Figure 6 presents in a schematic way an embodiment of the X-ray tube for use in the mobile X-ray device

### DETAILED DESCRIPTION OF THE DRAWINGS

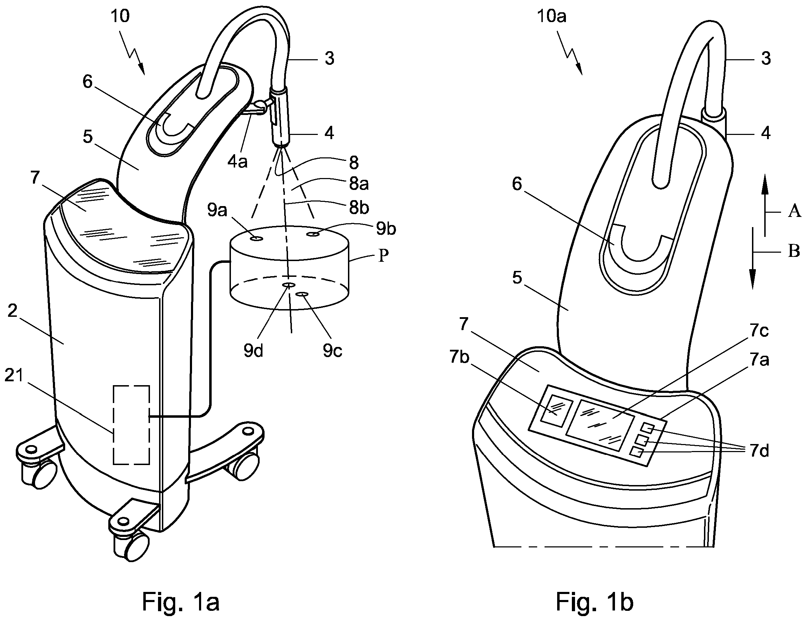

Figure 1a presents in a schematic way an embodiment of a mobile X-ray unit. The mobile X-ray unit 10 comprises a base 2 comprising at least a power supply unit, a cooling system and a control unit for controlling an operation of the X-ray applicator 4 comprising an X-ray tube accommodated in an outer housing. The X-ray applicator 4 is connected with the base using flexible cables 3, which may be at least partially received in a displaceable panel 5. The applicator 4 is supported by an articulated displaceable arm 4a, which may comprise a pivot for altering angulation of the applicator 4 in space. The applicator 4 comprises a longitudinal axis and an exit window 8 through which the generated X-ray beam emanates. The articulated arm 4a may also be mechanically connected with the displaceable panel 5 for enabling alteration of a vertical position of the X-ray applicator 4. Preferably, the displaceable panel 5 is provided with a handle 6 enabling easy manipulation thereof. The displaceable panel 5 may be guided along suitable rails for enabling a substantially smooth and shock-free displacement thereof.

Preferably, the X-ray applicator accommodating the X-ray tube has coaxial geometry, wherein the X-ray beam 8a conceived to irradiate a target region on a surface P' of a person P propagates from the exit window 8 having a beam axis 8b substantially corresponding to the axis of the X-ray tube. This can be enabled, for example, by arranging a longitudinal axis of an anode of the X-ray tube substantially parallel to a longitudinal axis of the X-ray applicator 4.

In accordance with an aspect, a phantom-base dosimetry system 9a, 9b is provided for providing data on at least the percentage depth dose of the emanating X-ray field. Preferably, for the dosimetry system 9a, 9b a system capable of generating on-line data is selected. Ionization chambers and solid state detectors, for example semiconductor detectors are suitable for this purpose. Such detectors may be arranged on the surface of the phantom-based dosimetry system as well as at specific depth, for example at 5 mm depth. By providing a plurality of point measurements enabled by the radiation dose meters parameters such as the percentage depth dose and beam flatness may be determined. It will be appreciated that the dosimetry system may be operable to provide relative data. For absolute dosimetry a calibration may be carried out. For determining percentage depth dose a number of dose meters 9d (at least two) at specific depths on the central axis 8b may be provided. For soft X-rays (50 - 130 kV) it is sufficient to measure data at the surface and at 5 mm depth. It will be further appreciated that the set of dose meters may be as large as 10 - 20 or even up to 50 detectors. For preventing their mutual interference in the phantom-based dosimetry system, the dose meters located at depth 9c are preferably positioned outside the shadow of the respective dose meters located at the surface 9b. Preferably, the signal from the dosimetry unit is supplied into the control unit 21 of the X-ray apparatus for on-line dose delivery control and/or interrupt.

Preferably, for positioning the X-ray applicator 4 and the dosimetry system 9a with respect to each other the applicator is provided with an indicator arranged to visually delineate the X-ray field to be generated by the X-ray tube inside the applicator 4. Preferably, the indicator comprises a light source, such as a light emitting diode, a laser or the like.

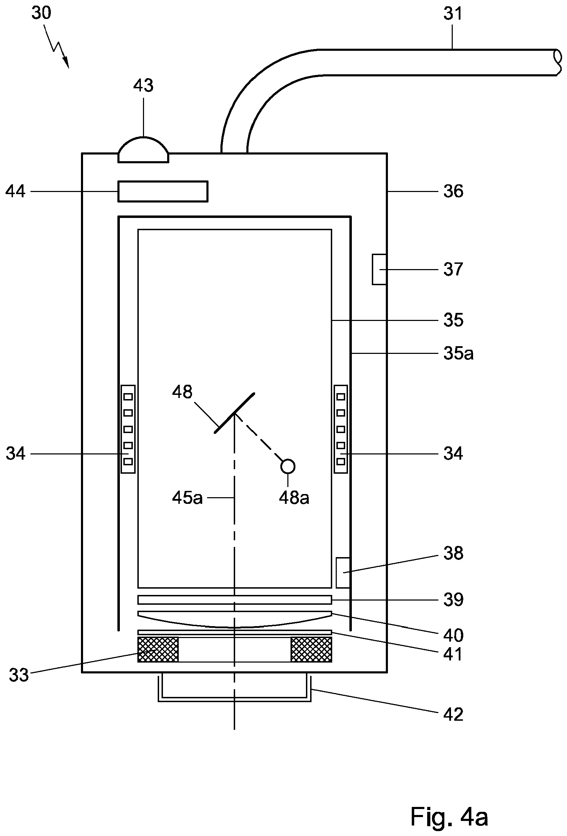

The light source may be arranged either inside the X-ray applicator 4, or around the X-ray applicator, or it may be remotely positioned, for example in the base 2. In the latter case light from the light from the light source (not shown) may be conducted towards the X-ray applicator using suitable one or more optical fibers. More details on the indicator will be presented with reference to Figures 4a - 4c.

Preferably, in the X-ray unit 10 a base unit 2 is provided with a display 7 for feeding-back suitable user information. The display 7 may be arranged as a touch-sensitive screen for enabling suitable data input into the system. For example, the display panel may comprise mean for switching the light indicator on. Optionally, the light indicator may always be on when the X-ray unit is switched on. The user interface may further be used to input prescribed dose and, possibly, prescribed dose distribution, especially when dose modifiers are used for intruding a gradient in the dose profile across the X-ray field. The user interface may also be arranged to display data on actual dose delivery and dose distribution profile during the treatment. It will be appreciated that by using the dosimetry system the dose delivery protocol may be compared with actual dose delivery data and, if necessary, the actual dose delivery may be corrected during further subsequent sessions should a discrepancy in prescribed and delivered dose of more than 1% occur.

Figure 1b presents in a schematic way an embodiment of a displaceable panel of the mobile X-ray unit. In this enlarged view 10a specific elements of the displaceable panel 5 are depicted. Accordingly, a handle 6 may be implemented as a mechanical item for pulling or pushing the panel 5. Alternatively, the handle 6 may be arranged as an electrical actuator for triggering motors (not shown) for displacing the panel 5. For example, when the handle 6 is pulled the motors may be activated for causing the panel 5 to displace in direction A. Pushing of the handle 6 may cause lowering of the panel 5 in direction B. Preferably, the mobile X-ray unit comprises means for limiting a travel distance of the panel 5. This may be advantageous for ensuring mechanical stability of the system on one hand (limitation of the upper level) and, on the other hand, may be beneficial for preventing cable damage (limitation of the lower level). Preferably, the panel 5 is movable using built-in rails whose length may be chosen for limiting the displacement range of the panel 5 in a desirable way.

The base unit 2 preferably further comprises a display 7, which may function as a suitable user interface 7a. For example, the patient data, such as a photo of the patient and/or a photo of a lesion may be provided in the window 7b, whereby relevant patient information, such as the date of birth, gender, dose prescription and dose delivery protocol and so on may be displayed in window 7c. Buttons 7d may be provided as touch functionality for enabling entering data. Alternatively or additionally, suitable hardware switches or buttons may be provided as well.

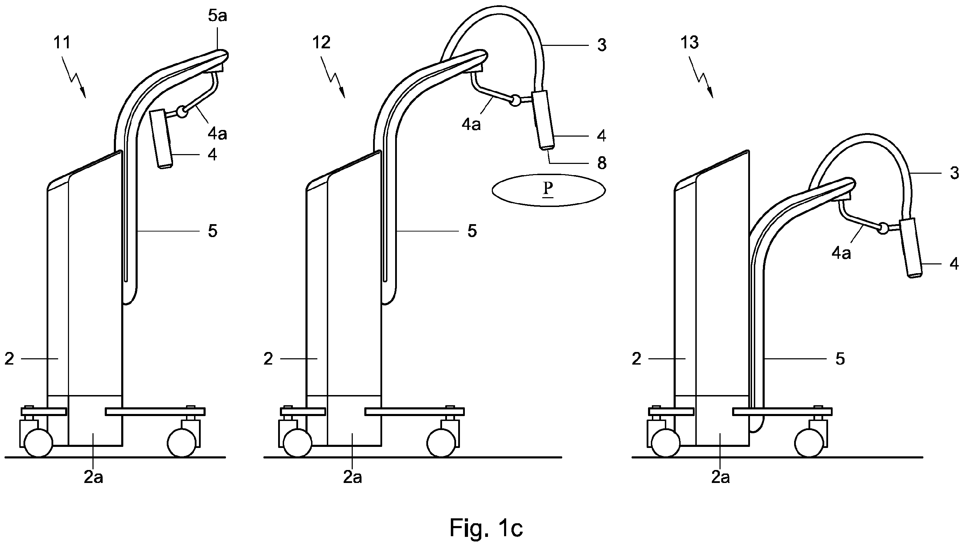

Figure 1c presents in a schematic way an embodiment of displacement functionality of the applicator in the X-ray unit according to the invention. In accordance with an aspect of the invention mechanics of the mobile X-ray unit is developed and realized to support a broad range of translational and rotational movements for the X-ray applicator 4.

In view 11 a schematic embodiment is presented wherein the X-ray applicator is in its parked position. It will be appreciated that cabling and optical fibers are not depicted for clarity reasons. Such position may be suitable for transport of the mobile X-ray unit towards a booth and/or for maneuvering the X-ray unit around the patient. In order to retract the X-ray applicator as close as possible to the base 2, the articulated arm 4a may be bent under the outer portion 5a of the displaceable panel 5. For ensuring stability of the mobile X-ray unit during maneuvering thereof, a load block 2a close to a floor is provided for lowering an absolute position of the center of gravity of the overall construction.

View 12 presents in a schematic way a further possibility, wherein the X-ray application 4 is in one of its working positions having an X-ray exit surface 8 being oriented towards a patient P. In order to suitably position the X-ray applicator with respect to the patient P, the displaceable panel may be moved to a certain dwell position located between a lowest position and a highest position of the panel 5. The articulated arm 4a may be used for suitably rotating the X-ray applicator about a rotation axis. Preferably, a rotation axis is selected to coincide with a direction of emanation of the X-ray beam from the exit surface when the X-ray tube is vertically oriented.

View 13 presents in a schematic way a still further possibility, wherein the X-ray applicator 4 is to be used at a lowered position. For this purpose the displaceable panel 5 may resume its lowest stand and the arm 4a may be used for orienting the X-ray applicator in a desirable way.

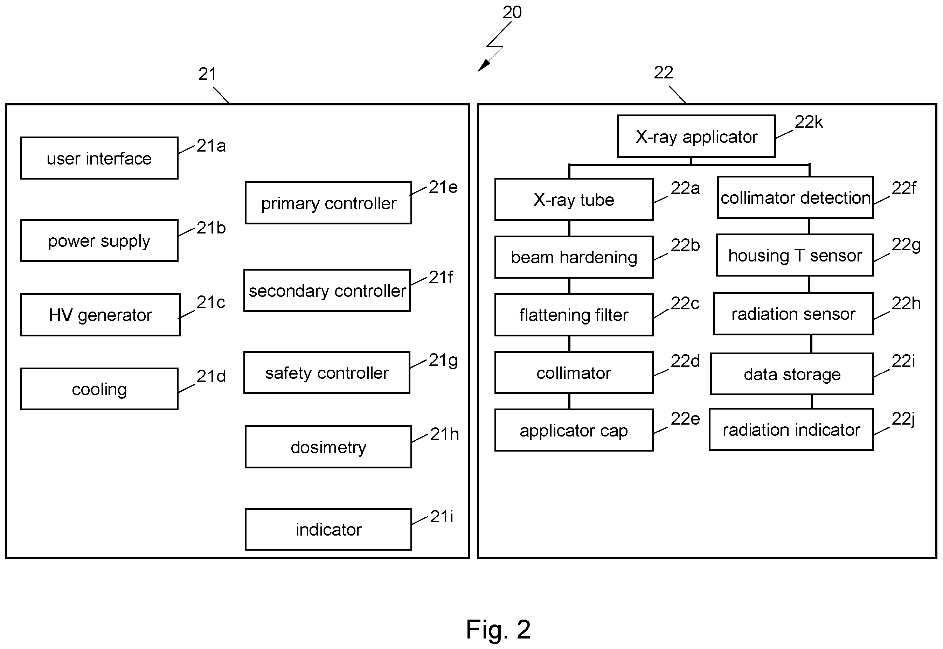

Figure 2 presents in a schematic way an embodiment of architecture of the mobile X-ray unit. The mobile X-ray unit comprises a high voltage power supply, preferably adapted to generate 50 - 75 kV X-rays in a suitable X-ray tube, a cooling system for cooling the X-ray tube during use and a control system for controlling electronic and electric parameters of sub-units of the X-ray unit during use. View 20 schematically depicts main units of the control system 21 and of the X-ray applicator 22. However, the X-ray tube may be also arranged to operate in 50 - 130 kV range.

The control system 21 preferably comprises a hard wired user interface 21a for enabling switching on and switching off of the high voltage supply 21b. Preferably, the high voltage supply 21b comprises a high voltage generator 21c with improved ramp-up and ramp-down characteristics. Preferably, the ramp-up time is of the order of 100 ms. The hard wired interface 21a, may also be arranged to automatically switch on the cooling system 21d upon an event the high voltage generator is switched on. In addition, the control system 21 may comprise a primary controller 21e arranged for controlling the dose delivery of the X-ray applicator in use. Such primary controller 21e may be provided with a primary counter adapted to register time lapsed after the X-ray radiation is initiated. The primary counter may then automatically switch off the high voltage supply to the X-ray tube upon an event a pre-determined dose is reached. It will be appreciated that the pre-determined dose is at least dependent on the energy of generated X-rays and the dose rate, wherein such dependence may be calibrated in advance. Provided corresponding calibrated data is made available to the primary controller adequate primary dose delivery control may be achieved. Preferably, a secondary controller 21f is provided for enabling an independent loop of dose delivery control. The secondary controller may be connected to a dose meter accommodated inside the X-ray applicator in the X-ray field before the collimator. Accordingly, the dose meter may provide real-time data on actual dose delivery taking into account dose variation during ramp up and ramp down of the high voltage source. Still preferably, the control system may further comprise a safety controller 21g adapted to compare readings from the primary controller 21e and the secondary controller 21f for triggering switching off of the high voltage generator 21c wherein a desired dose is delivered. In addition or alternatively, the safety controller 21g may be wired to guard emergency stop, door interlock and a generator interlock.

The control system may further comprise a dosimetry control 21h, adapted to communicate with the phantom-based dosimetry system, preferably on-line. However, it is also possible that the dosimetry control 21h may accept data about the analyzed dosimetric field and update dose delivery data using to such data.

The dosimetry control 21h is preferably arranged to provide an interrupt signal, should the on-line dosimeter measure a substantial deviation between the prescribed dose and the measured dose. For example, the dosimetry control 21h may provide a suitable interrupt signal to the high voltage generator control 21c.

The control system may further comprise an indicator controller 21i for controlling the light source for delineating at least a portion of the X-ray beam. Although for simplicity the indicator controller 21i may be linked to a power supply unit 21b for switching on the light source once the system is on, it is preferable, that the light source is switched on demand. Accordingly, the indicator control may be arranged to provide electrical power to the light source when triggered by the user. The user may provide a suitable trigger signal by means of a user interface, or, for example, using a dedicated hardware switch.

The X-ray applicator 22 may preferably comprise the following features: an X-ray tube 22a, conceived to be housed in an outer housing (shielding) 22k.

According to the invention the X-ray tube is provided having a coplanar target, collimator and the exit window geometry causing the generated X-ray beam to propagate substantially parallel to the longitudinal axis of the X-ray tube. Preferably, a target-collimator distance of about 4 - 10 cm, preferably about 5 to 6 cm. It will be appreciated that the target - collimator distance is quoted as a distance between the outer surface of the target plate and a midplane of the collimator. The X-ray applicator may further comprise a beam hardening filter 22b selected to intercept low-energy radiation and a beam flattening filter 22c, designed to intercept portions of X-ray radiation for generating a substantially flat beam profile near the exit surface of the X-ray applicator. Further, the X-ray applicator 22 may comprise one or more collimators arranged to define treatment beam geometry. Preferably a set of collimators is used, having diameters, for example, of 1, 1.5, 2, 2.5, 3, 3.5, 4, 4.5, 5 cm. It will be appreciated that although circular collimators are discussed, collimators of any shape, like square, elliptic or custom made are possible. It is found to be advantageous to provide the X-ray applicator 22 with automatic collimator detection means 22f adapted to automatically signal which collimator is being used. Preferably, resistive sensing is used, wherein each collimator is provided with at least a couple of projections for bridging a resistive path provided in a collimator receptacle. The resulting electrical resistance of the receptacle constitutes a signal representative of a collimator being used. The X-ray applicator 22 still further preferably comprises a built-in temperature sensor adapted to signal temperature of the X-ray tube and/or the outer housing (shielding). The signal from the temperature sensor is received by the control system which carried out analysis thereof. Should the measured temperature be elevated beyond an allowable level, an alarm signal may be generated. Optionally, a shut-off signal to the high voltage generator may be provided. The X-ray applicator 22 further comprises a radiation sensor 22h arranged inside the outer housing 22k for detecting X-ray radiation which is actually being delivered by the X-ray tube. Preferably, for safety reasons the X-ray applicator 22 further comprises a non-volatile data storage 22i arranged for recording operational parameters at least of the X-ray tube. Further, to enhance radiation safety, the X-ray applicator 22 may be provided with a radiation indicator 22j arranged for providing a visual and/or an audio output to the user and/or the patient regarding ON/OFF condition of the X-ray tube. It will be appreciated that the radiation indicator 22j may comprise a plurality of distributed signaling means. Preferably, at least one signaling means, for example a light emitting diode (LED) is associated with the X-ray applicator 22. More preferably, the signaling means is provided on the X-ray applicator 22.

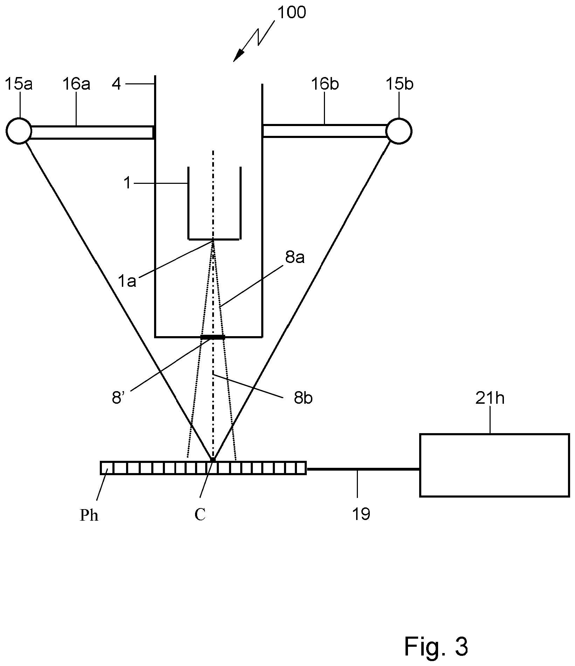

Figure 3 presents in a schematic way a phantom-based dosimetry system of the X-ray unit according to the invention. The phantom-based dosimetry system 100 is adapted to be positioned with a distance with respect to the X-ray applicator 1, as is representative for patient treatments. Usually the distance will be in the order of 0.5 - 2 cm. However, a distance of up to 10 cm may be envisaged. The phantom-based dosimetry system is preferably used to obtain dosimetry data for all representative distances between the X-ray applicator 1 and the phantom Ph. It will be appreciated that although the phantom Ph for simplicity reasons is shown as a flat structure, the phantom Ph may have a shape corresponding to a non-flat surface on a human body or an external organ. Such phantom may be referred to as a humanoid phantom. More preferably, the humanoid phantom has a Z value corresponding to that of a human soft tissue.

In order to align the phantom Ph with respect to the applicator 1, the former may be provided with an alignment means, and the latter may be provided with means for visualizing the X-ray beam. However, it may be sufficient to visualize only a central ray of the X-ray beam 8a, using a laser, for example.

The X-ray applicator 4 discussed with reference to the foregoing, comprises an X-ray tube arranged with an anode 1 having a target region 1a for generating a diverging X-ray beam 8a. The target region 1a is a substantially flat plate which extends substantially perpendicular to the longitudinal axis of the anode 1. Although preferably the anode 1 is oriented coaxially with the axis 8b of the X-ray beam (and the X-ray tube), other respective orientations are possible. The generated X-ray beam is emitted by the X-ray applicator from an exit surface 8'. It will be appreciated that suitable filters, a collimator and an exit window of the X-ray tube are not depicted for clarity reasons. Accordingly, the exit surface 8' does not necessarily correspond to the exit window of the X-ray tube.

Preferably, for positioning the X-ray applicator 4 with respect to a target region of the patient, an indicator is used. The indicator may comprise two light sources 15a, 15b arranged to generate a narrow beam light, said light sources being mounted on respective support arms 16a, 16b and by their means to the outer surface of the X-ray applicator 4. Preferably, the light sources are arranged to provide a point in space C corresponding to the beam axis 8b. The dosimetry system Ph may then be centered with respect to the point C for intercepting the X-ray beam.

In order to obtain percentage depth dose data the phantom Ph may be provided with a plurality of dose meters, distributed within its volume. For example, a number of dosimetric films may be provided across the X-ray field at characteristic depths, like, for example at 5 mm, at 1 cm and at 5 cm. Such transversal films may also be used to examine beam flatness at specified depths.

Alternatively, a dosimetric film extending in a vertical plane of the phantom Ph and running through a central axis 8b may be provided. Such arrangement may be used for providing continuous depth dose data.

It will be appreciated that the phantom Ph may be used in a great variety of ways cooperating with a plurality of radiation detectors which are contemporary available to carry out dosimetry of soft X-rays. Those skilled in the art readily appreciate corresponding embodiments of the phantom Ph.

Preferably, the phantom-based dosimetry system 100 provides on line reading, which may be provided using suitable cabling 19 to the dosimetry control unit 21h, as discussed with reference to Figure 2. It will be appreciated that the cabling 19 may be used in combination with an electronic dose meter, such as an ionization chamber or a semiconductor detector.

Although an embodiment of the dosimetery system is discussed with reference to the X-ray applicator provided with the field delineation means, it will be appreciated that the invention may be practiced when no indicator delineating the X-ray field is provided.

Figure 4a presents in a schematic way a first embodiment of a cross section of an X-ray applicator of the mobile X-ray unit depicting a first embodiment of the indicator for delineating the X-ray field emanating from the X-ray applicator. The X-ray applicator 30 comprises an outer housing 36 accommodating the X-ray tube assembly 35 provided with external shielding 35a.

The X-ray applicator 30 may further comprise a light source 48a cooperating with a mirror 48 for emitting a light a beam indicative of a beam of X-rays produced by the X-ray tube. Preferably, X-rays have a propagation axis 45a which coincides with a longitudinal axis of the X-ray tube. The light source 48a and the mirror 48 are arranged to cause the generated light beam to propagate substantially along the longitudinal axis of the X-ray tube assembly 45a.

When the thus formed light beam is intercepted by the collimator 33 a visual indication of the X-ray beam is produced facilitating accurate alignment between the X-ray applicator and the target area of the patient.

Preferably, the distance between the target (anode) and the collimator 33 is in the range of 4 ... 10 cm, preferably about 5 to 6 cm. Such relatively short target-collimator distance is advantageous for generating an X-ray beam having a substantially narrow penumbra (1.5 - 1.8 mm for 20/80% lines) and good beam flatness.

The X-ray applicator 30 further comprises a filter 39 for hardening the X-ray beam emanating from the target 45, a beam flattening filter 40 for flattening out a beam profile and collimator 33 insertable in a collimator receptacle 41.

In order to prevent overheating of the X-ray tube in use a cooling system 34 is provided, which may advantageously be arranged in spacing between the X-ray tube 35 and the shielding 35a in contact with the surface of the X-ray tube 35. A suitable coolant may be provided using a pipe 31. Preferably, the coolant is circulating and may refer to water or pressurized gas. The X-ray applicator may comprise a temperature sensor 37.

The X-ray assembly 30 may further comprise a suitable radiation detector 38, connected to a radiation indicator 43. Preferably, data collected by the radiation detector 38 is stored in a data storage unit 44.
In order to protect an X-ray exit surface of the X-ray applicator 30 from intra-patient contamination, an applicator cap 42 may be provided to cover at least the exit surface of the X-ray applicator 30. Preferably, the applicator cap is thick enough to fully intercept secondary electrons emanating from the X-ray applicator. Preferably, the applicator cap is manufactured from PVDF (Polyvinylidene fluoride) and is about 0.4 - 0.7mm, preferably 0.6 mm thick across the window portion, having density of about 1.75 - 1.8, preferably 1.78. Alternatively the applicator cap may be 0.3 - 0.6mm, preferably 0.5 mm thick across the window portion and having density of 1.30 - 1.45, preferably 1.39, being manufactured from PPSU (polyphenylsulfone). It is found that these materials are particularly suitable as they as stable under influence of the X-rays and are suitable for different types of sterilization procedures, such as chemical sterilization, or sterilization under elevated temperatures.

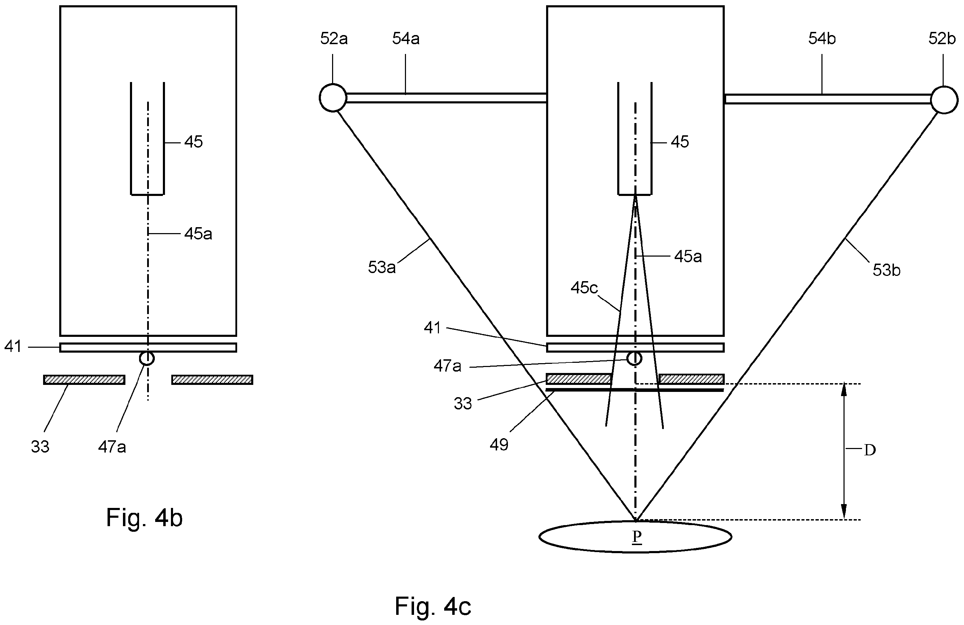

Figure 4b presents in a schematic way a second embodiment of a cross section of an X-ray applicator of the mobile X-ray unit depicting a second embodiment of the indicator. In this exemplary embodiment an optical fiber 47a is provided in the collimator receptacle 41 above the collimator 33. The optical fiber 47a is arranged to generate a light field being substantially centered about the collimator opening 33 for simulating an X-ray beam emanated from the collimator. For this purpose the optical fiber 47a is arranged to emit a substantially narrow beam having divergence representative with expected divergence of the X-ray beam.

Alternatively, it is possible to use the optical fiber 47a for visualizing a central axis 45a of the X-ray beam. In this case the optical fiber is advantageously arranged to emit a narrow beam light producing a miniature light spot on a surface of the patient. Preferably, a dimension of the light spot is less than 5 mm², more preferably a dimension of the light spot is about 1 mm². A suitable light emitting diode or a laser may be used for generating light emanating from the fiber 47a. Preferably, the light emitting diode and the laser are remotely arranged with respect to the X-ray applicator 30. It will be appreciated that an alternative configuration may be used wherein one or more light sources cooperate with one or more optical fibers.

Figure 4c presents in a schematic way a third embodiment of a cross section of an X-ray applicator of the mobile X-ray unit depicting a third embodiment of the indicator. In this particular embodiment the X-ray applicator having a target 45 for generating an X-ray beam 45c having the longitudinal X-ray axis 45a is provided with external indicator for visualizing the longitudinal axis 45a at a pre-determined distance D from the lower surface 49 of the X-ray applicator. It will be appreciated that the lower surface 49 may relate to the exit window as discussed with reference to Figure 1c, or it may relate to the applicator cap as is discussed with reference to Figure 4.

The external indicator comprises one or more light sources 52a, 52b disposed on respective support arms 54a, 54b for generating respective narrow light beams 53a, 53b said beam being directed towards the axis 45a and being adapted to intersect at the pre-determined distance D from the lower surface 49 of the X-ray applicator 30. Preferably, the Distance D is selected to be between 0.5 and 2 cm. The support arms 54a, 54b are arranged in such a way that the light beams 53a, 53b do not intercept the X-ray applicator.

When positioning the X-ray applicator with respect to the patient P, the former must be maneuvered in such a way that the beams 53a, 53b intersect at the surface of the patient. However, should the treatment regime assume use of a dose build-up material, the beams 53a, 53b may cross on a surface of the dose build-up material. Preferably, the support arms 54a, 54b are adjustable for enabling indication of the central axis 45a at different distances from the lower surface 49 of the X-ray applicator.

In order to calibrate adjustment of the support arms, a transparent calibration phantom may be used, wherein the central axis and depth are marked. It will be appreciated that although Figures 4a - 4c disclose separate embodiments of the indicator, combination of such embodiments is contemplated as well. For example, means for indicating the central axis may be combined with means for indicating the complete field. In addition, internal and external indicators may be combined as well.

Figure 5a presents in a schematic way an embodiment of a phantom-based dosimetry system according to an aspect of the invention. According to the present embodiment the X-ray applicator 4 and the phantom-based dosimetry system are adapted to form an affixed configuration 60. For example, the phantom-based dosimetry system may be screwed, clicked, slide, or otherwise affixed to the X-ray applicator 4. In the present embodiment the phantom-based dosimetry system 61 comprises a handle 62 for handling the components. The X-ray applicator 4 or the phantom-based dosimetry system may be provided with a suitable body 64 cooperating with a suitable number of screws 67a, 67b for firmly attaching the dosimetry system to the X-ray applicator. The body 63 of the phantom-based dosimetry system may be implemented from a plastic material, or, preferably, from a tissue equivalent material.

It is found to be advantageous to provide the phantom-based dosimetry system which can be attached to the X-ray applicator 4, as suitable dosimetric measurements may be carried out for different angulations of the X-ray applicator 4.

Figure 5b presents in a schematic way an embodiment of the phantom-based dosimetry system of Figure 5a in a cross-section. As follows from Figure 5a, the X-ray applicator 4 is delivering a beam of the X-ray radiation 8a, which is arranged to impinge on the phantom-based dosimetry system 61. It will be appreciated that the phantom-based dosimetry system 61 may comprise a background material 63 and a similar or a different material 66 used for accommodating a suitable set of detector elements. For example, a PCB material may be used for accommodating the radiation detectors.

It will be appreciated that an embodiment, wherein the radiation detectors provided in the phantom-based dosimetry system are adapted to provide a signal representative of the measured radiation dose is contemplated. For example, the background material 63 may comprise a transmitter 68 electrically connected to the detectors and adapted for wirelessly transmitting the corresponding read-out signals. It will be appreciated that the transmitter 68 may be provided at any desirable place on or inside the phantom, preferably outside the X-ray beam. Alternatively, the phantom may be provided with an electronic device, which may be connected using suitable cables to an external data acquisition or data processing unit. Those skilled in the art will readily appreciate how to implement the described embodiments of the data communication unit.

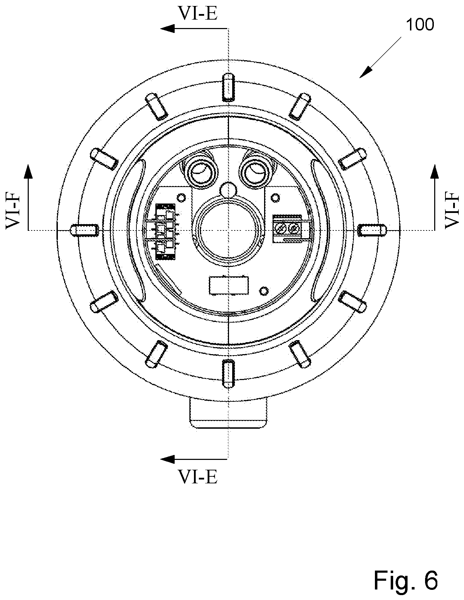

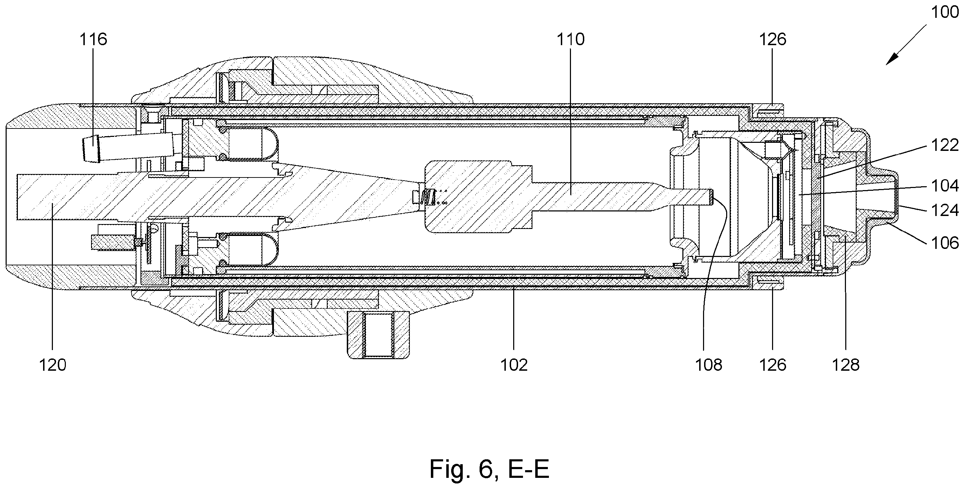

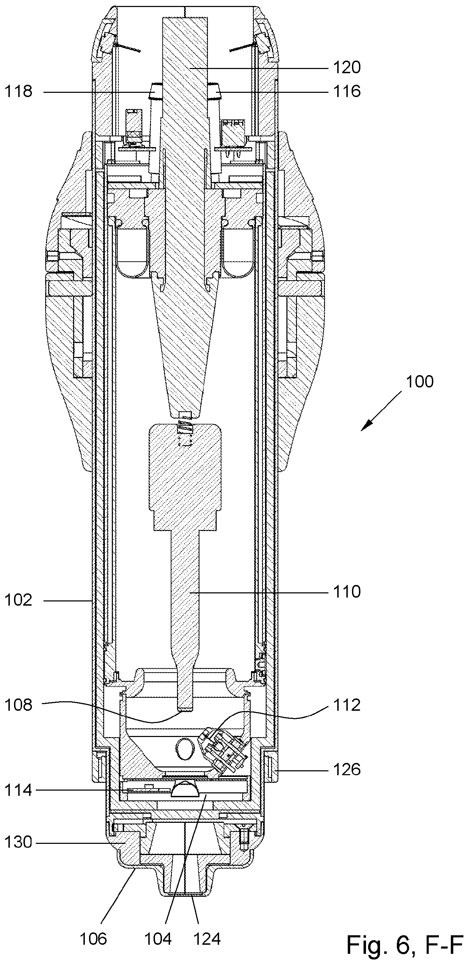

Figure 6 presents in a schematic way an embodiment of the X-ray tube for use in the mobile X-ray device. The X-ray tube 100, has a body 102 enclosing at one end an end window 104 through which the X-rays pass. The end window is made from a thin sheet of Beryllium metal. Covering the end window 104 to provide protection against the damage of the window and protection against the toxic effects of the metal is an applicator cap 106. Applicator cap 106 is preferably made from a plastic material.

In the tube body 102 a target 108 is located at between 4 -10 cm from a collimator 130, and preferably at 4-6cm from the collimator 130 (see Figure 6, cross-section F-F). The distance is quoted as a distance between the outer surface of the target plate and midplane of the collimator 130. The target is made from Tungsten metal to provide the desired X-ray spectrum. The tungsten tip of the target is mounted on a large anode assembly 110 which also serves to conduct away the heat created from the generation of the X-rays in the target. Most of the anode assembly is made from copper. The cathode 112 is located slightly off-axis near the end window. Electrons emitted from the cathode are accelerated across the gap by the potential difference between the cathode and anode, in this case set at about 70kV, to the target which they impact and cause the generation of X-rays in a known manner. X-rays emitted from the target 108 pass through a beam hardening filter 122 before passing through a collimator 130 and an exit surface 124 on an applicator cap 106. The collimator 130 may be housed in a suitable collimator receptacle 128.

The anode assembly 110 is mounted in the body 102 and electrically insulated from it. One of a number of known techniques and materials can be used to provide the desired level of insulation between the anode and the body 102.

As is also well known in the art, the production of X-rays generates large amounts of waste heat, with the result that it is necessary to cool the tube in order to maintain it at a safe temperature. Various cooling mechanisms are known and used in the art. In this embodiment, the tube is cooled by means of water forced around the anode region. Water enters the back of the tube by means of conduits 116 and leaves by means of a second conduit 118. The water cooling circuit is a closed loop circuit, with the water leaving the tube assembly to be cooled by a remote cooler (not shown) before returning to the tube. Alternatively oil or another liquid could be used as the cooling medium. It is also known that a pressurized gas is used as an effective coolant in some applications.

As is known in the art, X-rays are generated and emitted in all directions, but the shielding by the body of the tube 102 and other internal components will tend to reduce the amount of radiation emitted from the body of the tube to a minimum, with most of the radiation emitted from the end window. The thickness of the shielding provided by the body is designed such that it provides at least the minimum level of shielding required for safe use by the operator.

A high voltage cable assembly 120 is connected to the anode assembly 110. The high voltage cable assembly is connected to flexible cable means (not shown) which in turn is connected to a high voltage power supply.

A radiation detector 114 is placed outside the path of the X-ray beam emitted from the target 108 and passing through the end window 104. This detector can be any known form of radiation detector. In this embodiment it is a known form of suitably radiation hardened semi-conductor connected to an amplifier. The radiation detector 114 detects when the tube 102 is working and emitting X-ray energy. Output from the detector is connected to a control unit, the output signals from which may be used to provide an optical indication of whether the tube is operating or not. By this means an X-ray detector is provided which can be used to detect whether the tube is on or off.

With further calibration of the radiation detector 114, it is possible to determine and calculate the X-ray dose administered to the patient during the treatment. By this means it is possible to have a real time dosimetry measurement system, in which the precise amount of radiation dose administered can be determined. Once the dose rate is known, a treatment plan can be modified during treatment. This is advantageous because it enables a very accurate and carefully controlled dose of X-rays to be administered.

In order to enable the tube 102 to be placed accurately over a tumour, a tumour illumination means is used. The tumour illumination means comprises a plurality of lights 126 placed around the circumference of the tube near the end window. When in use, the lights shine onto the skin of the patient. Since the lights 126 are positioned around the circumference of the tube body 102, at a short distance from the end of the tube they create a circle of light with a sharp cut off of the inner part of the circle. In this way, the position of the lights on the tube body 102 creates a shadow. This shadow circle is used to indicate the region which will be subject to irradiation when the X-ray tube is turned on. It should be appreciated the area within the circle will not be completely dark; the ambient light will be able to enter the shadow region.

Preferably the lights 126 are white LEDs which can be bright enough to clearly illuminate the target region but do not generate amounts of heat and have very long lives. The lack of heat generation is important because the lights will be in close proximity to the skin of the patient, and so it is important to minimise the risk of burning or other damage to the skin. Other colours of LEDs could be used. Alternatively, other light sources could be used, such as known filament lamps or even a remote light source connected to the ring by fibre optic cables.

It will be further appreciated that when a reference to an on-line dosimeter is made, a real-time functionality is contemplated as well. The descriptions above are intended to be illustrative, not limiting. The invention is defined by the appended claims.

## Claims

1. A mobile X-ray unit comprising
a base (2) for accommodating a control unit (21) and a power supply (21b), further comprising
an articulated displaceable arm (4a) supporting an X-ray applicator (4) having an X-ray tube (22, 35, 100) for emitting an X-ray beam through a collimator (22d) and an exit window (8) causing the generated X-ray beam to propagate parallel to the longitudinal axis of the X-ray tube (22, 35, 100), for irradiating an object, wherein the X-ray unit further comprises
a phantom-based dosimetry system (9a, 9b, 61, 100) including a body (63) of tissue equivalent material, the dosimeter system being screwed, clicked or slide to the X-ray applicator (4) to form an affixed formation (60); and being adapted to perform a real-time dosimetry check of the X-ray beam.

2. The mobile X-ray unit according to claim 1, wherein the dosimetry system (9a, 9b, 61, 100) comprises digital read out means.

3. The mobile X-ray unit according to claims 1 or 2, wherein the dosimetry system (9a, 9b, 61, 100) is arranged to electronically communicate to the control unit (21).

4. The mobile X-ray unit according to any one of the preceding claims, wherein the dosimetry system (9a, 9b, 61, 100) is arranged to enable verification of at least a percentage depth dose, a beam flatness and a dose rate of a generated X-ray field or dose variation during ramp up and ramp down of the high voltage source.

5. The mobile X-ray unit according to any one of the preceding claims, wherein the dosimetry system (9a, 9b, 61, 100) is calibrated for enabling absolute dosimetry of the percentage depth dose.

6. The mobile X-ray unit according to any one of the preceding claims, wherein the phantom comprises one or more dose meters (9d) located at pre-determined positions inside the phantom.

7. The mobile X-ray unit according to claim 6, wherein the phantom is manufactured from a solid material.

8. The mobile X-ray unit according to any one of the preceding claims, wherein a surface of the phantom is provided with an alignment pattern.

9. The mobile X-ray unit according to any one of the preceding claims, wherein the dosimetry system (9a, 9b, 61, 100) is arranged to be attached to a receptacle of the X-ray applicator (4).

10. The mobile X-ray unit according to any one of the preceding claims, wherein the X-ray unit further comprises an indicator for providing a visual indication of at least a portion of the X-ray beam emitted from the exit window (8).

11. The mobile X-ray unit according to any one of the preceding claim, wherein a radiation detector is provided inside the X-ray applicator (4) for detecting the X-ray beam.

12. The mobile X-ray unit according to claim 11, wherein the radiation detector is arranged to generate a control signal upon generation of the X-ray beam.

13. The mobile X-ray unit according to any one of the preceding claims, wherein the said phantom is humanoid.

14. A method for dosimetry control of an X-ray beam emanating from a mobile X-ray unit according to claim 1, the method comprising the step of:
- providing the phantom-based dosimetry system (9a, 9b, 61, 100) for verifying at least a percentage depth dose of the X-ray beam.

15. The method according to claim 14, wherein a solid phantom provided with one or more dose meters (9d) is used.

## Patentansprüche

1. Eine mobile Röntgeneinheit, umfassend:
eine Basis (2) zur Aufnahme einer Steuereinheit (21) und einer Stromversorgung (21b), ferner umfassend:
einen schwenkbaren beweglichen Arm (4a), der einen Röntgenapplikator (4) mit einer Röntgenröhre (22, 35, 100) trägt, um einen Röntgenstrahl durch einen Kollimator zu emittieren (22d);
und ein Austrittsfenster (8), das bewirkt, dass sich der erzeugte Röntgenstrahl parallel zur Längsachse der Röntgenröhre (22, 35, 100) ausbreitet, um ein Objekt zu bestrahlen, wobei die Röntgeneinheit ferner umfasst:
ein phantombasiertes Dosimetriesystem (9a, 9b, 61, 100), das einen Körper (63) aus gewebeäquivalentem Material enthält, wobei das Dosimetriesystem mit dem Röntgenapplikator (4) verschraubt, geklickt oder geschoben wird, um eine befestigte Formation zu bilden (60); und angepasst ist, um eine Echtzeit-Dosimetrieprüfung des Röntgenstrahls durchzuführen.

2. Mobile Röntgeneinheit nach Anspruch 1, wobei das Dosimetriesystem (9a, 9b, 61, 100) digitale Auslesemittel umfasst.

3. Mobile Röntgeneinheit nach Anspruch 1 oder 2, wobei das Dosimetriesystem (9a, 9b, 61, 100) so angeordnet ist, dass es elektronisch mit der Steuereinheit (21) kommuniziert.

4. Mobile Röntgeneinheit nach einem der vorhergehenden Ansprüche, wobei das Dosimetriesystem (9a, 9b, 61, 100) so angeordnet ist, das es die Überprüfung wenigstens einer prozentualen Tiefendosis, einer Strahlebenheit und einer Dosisrate eines erzeugten Röntgenfeldes oder einer Dosisänderung während des Anstiegs und Abfalls der Hochspannungsquelle ermöglicht.

5. Mobile Röntgeneinheit nach einem der vorhergehenden Ansprüche, wobei das Dosimetriesystem (9a, 9b, 61, 100) kalibriert ist, um eine absolute Dosimetrie der prozentualen Tiefendosis zu ermöglichen.

6. Mobile Röntgeneinheit nach einem der vorhergehenden Ansprüche, wobei das Phantom einen oder mehrere Dosismesser (9d) umfasst, die sich an vorbestimmten Positionen innerhalb des Phantoms befinden.

7. Mobile Röntgeneinheit nach Anspruch 6, wobei das Phantom aus einem festen Material hergestellt ist.

8. Mobile Röntgeneinheit nach einem der vorhergehenden Ansprüche, wobei eine Oberfläche des Phantoms mit einem Ausrichtungsmuster versehen ist.

9. Mobile Röntgeneinheit nach einem der vorhergehenden Ansprüche, wobei das Dosimetriesystem (9a, 9b, 61, 100) so angeordnet ist, dass es an einem Behälter des Röntgenapplikators (4) angebracht ist.

10. Mobile Röntgeneinheit nach einem der vorhergehenden Ansprüche, wobei die Röntgeneinheit ferner einen Indikator zum Bereitstellen einer visuellen Anzeige von wenigstens einem Teil des vom Austrittsfenster (8) emittierten Röntgenstrahls umfasst.

11. Mobile Röntgeneinheit nach einem der vorhergehenden Ansprüche, wobei ein Strahlungsdetektor innerhalb des Röntgenapplikators (4) zum Erfassen des Röntgenstrahls vorgesehen ist.

12. Mobile Röntgeneinheit nach Anspruch 11, wobei der Strahlungsdetektor so angeordnet ist, dass er bei Erzeugung des Röntgenstrahls ein Steuersignal erzeugt.

13. Mobile Röntgeneinheit nach einem der vorhergehenden Ansprüche, wobei das Phantom humanoid ist.

14. Verfahren zur Dosimetriesteuerung eines Röntgenstrahls, der von einer mobilen Röntgeneinheit nach Anspruch 1 ausgeht, wobei das Verfahren den Schritt umfasst:
- Bereitstellen des phantombasierten Dosimetriesystems (9a, 9b, 61, 100) zur Überprüfung wenigstens einer prozentualen Tiefendosis des Röntgenstrahls.

15. Verfahren nach Anspruch 14, wobei ein festes Phantom mit einem oder mehreren Dosismessern (9d) verwendet wird.

## Revendications

1. Unité radiologique mobile comprenant :
une base (2) destinée à recevoir une unité de commande (21) et une alimentation électrique (21b), comprenant en outre
un bras mobile articulé (4a) portant un applicateur radiologique (4) ayant un tube à rayons X (22, 35, 100) pour émettre un faisceau de rayons X à travers un collimateur (22d) et une fenêtre de sortie (8) amenant le faisceau de rayons X généré à se propager parallèlement à l'axe longitudinal du tube à rayons X (22, 35, 100), pour irradier un objet, dans laquelle l'unité radiologique comprend en outre
un système dosimétrique basé sur un fantôme (9a, 9b, 61, 100) incluant un corps (63) de matière équivalente à un tissu, le système de dosimètre étant vissé, encliqueté ou coulissé sur l'applicateur radiologique (4) pour former une formation apposée (60) ; et étant conçu pour réaliser une vérification dosimétrique en temps réel du faisceau de rayons X.

2. Unité radiologique mobile selon la revendication 1, dans laquelle le système dosimétrique (9a, 9b, 61, 100) comprend des moyens de lecture numériques.

3. Unité radiologique mobile selon la revendication 1 ou 2, dans laquelle le système dosimétrique (9a, 9b, 61, 100) est conçu pour communiquer de manière électronique avec l'unité de commande (21).

4. Unité radiologique mobile selon l'une quelconque des revendications précédentes, dans laquelle le système dosimétrique (9a, 9b, 61, 100) est conçu pour permettre la vérification d'au moins un pourcentage de dose en profondeur, une planéité de faisceau et un débit de dose d'un champ de rayons X généré ou une variation de dose au cours d'une intensification et d'une baisse de la source de haute tension.

5. Unité radiologique mobile selon l'une quelconque des revendications précédentes, dans laquelle le système dosimétrique (9a, 9b, 61, 100) est étalonné pour permettre une dosimétrie absolue du pourcentage de dose en profondeur.

6. Unité radiologique mobile selon l'une quelconque des revendications précédentes, dans laquelle le fantôme comprend un ou plusieurs dosimètres (9d) situés à des positions prédéterminées à l'intérieur du fantôme.

7. Unité radiologique mobile selon la revendication 6, dans laquelle le fantôme est fabriqué à partir d'un matériau solide.

8. Unité radiologique mobile selon l'une quelconque des revendications précédentes, dans laquelle une surface du fantôme est munie d'un motif d'alignement.

9. Unité radiologique mobile selon l'une quelconque des revendications précédentes, dans laquelle le système dosimétrique (9a, 9b, 61, 100) est conçu pour être attaché à un réceptacle de l'applicateur radiologique (4).

10. Unité radiologique mobile selon l'une quelconque des revendications précédentes, dans laquelle l'unité radiologique comprend en outre un indicateur pour fournir une indication visuelle d'au moins une partie du faisceau de rayons X émis par la fenêtre de sortie (8).

11. Unité radiologique mobile selon l'une quelconque des revendications précédentes, dans laquelle un détecteur de rayonnement est ménagé à l'intérieur de l'applicateur radiologique (4) pour détecter le faisceau de rayons X.

12. Unité radiologique mobile selon la revendication 11, dans laquelle le détecteur de rayonnement est conçu pour générer un signal de commande lors de la génération du faisceau de rayons X.

13. Unité radiologique mobile selon l'une quelconque des revendications précédentes, dans laquelle ledit fantôme est humanoïde.

14. Procédé de commande dosimétrique d'un faisceau de rayons X émanant d'une unité radiologique mobile selon la revendication 1, le procédé comprenant l'étape de :
- la fourniture du système dosimétrique basé sur un fantôme (9a, 9b, 61, 100) pour vérifier au moins un pourcentage de dose en profondeur du faisceau de rayons X.

15. Procédé selon la revendication 14, dans lequel un fantôme solide muni d'un ou de plusieurs dosimètres (9d) est utilisé.
